# EUROPEAN PATENT APPLICATION

(11) **EP 1 103 602 A1**
(43) Date of publication of application: **30.05.2001**
(21) Application number: 99120484.3
(22) Date of filing: 15.10.1999
(51) Int. Cl.: C12N 5/08, A61K 39/00, A61P 35/00

(54) **New lymphocytes, a process for preparing the same and their use, in therapeutics**

(71) Applicant: I.D.M. IMMUNO-DESIGNED MOLECULES, 75011 Paris (FR)
(72) Inventor: Abastado, Jean Pierre, 75015 Paris (FR); Bartholeyns, Jacques Manoir de la Vignole, 49730 Turquant (FR)
(74) Representative: Grosset-Fournier, Chantal Catherine

(57) **Abstract**

The invention relates to lymphocytes, having the property of recognizing cells of a patient in which the pericellular membrane expression of HSP or presentation of HSP epitopes has been induced and increased with respect to the normally occurring HSP expression level in the patient's body, and corresponding to an overexpression of HSP, particularly at a level of at least about 10 times the normally occurring level.

These lymphocytes are used, for instance, as active substance in pharmaceutical compositions

## Description

The invention relates to new lymphocytes, a process for preparing the same and their use in therapeutics.

More precisely, the invention relates to a new method of therapeutic vaccination against most forms of cancer or chronic infectious diseases.

Strivastava (US Patent 5 830 464) has demonstrated that HSPs (heat shock proteins) and particularly HPS70 extracted from tumors can be injected in a form of complex with tumor protein/peptide and do elicit an immune response against these autologous tumors. However, HSPs have a very conserved sequence and are not immunogenic as such, but are carriers of antigenic peptides that do elicit a specific immunity against the said tumor.

HSPs are supposed to be nonpolymorphic molecules that do not differ in their primary structure among normal tissues and cancers or among normal and virusinfected cells. It has thus been reported that the immunizing ability of HSP preparations is related to the association of HSP molecules with peptides generated in the cells from which the HSPs are isolated. Indeed, HSP-peptide complexes can be generated *in vitro,* and the biological activity of these complexes is comparable with that of HSP-peptide complexes generated *in vivo* (Blachere, N.E.et al 1997; Heat shock protein-peptide complexes reconstituted *in vitro,* elicit peptide-specific cytotoxic T lymphocytes response and tumor immunity. J. Exp.Med. 186-1315). HSP 70 has also been used with CTL epitopes to induce efficient protective antiviral immunity in addition to the generation of peptide-specific CTLs, and therefore represents an alternative to adjuvant and DNA vectors for the delivery of CTL epitopes to APCs (Ciupitu A.M. et al 1998; Immunization with a lymphocytic choriomeningitis virus peptide mixed with heat shock protein 70 results in protective antiviral immunity and specific cytotoxic T. lymphocytes. J; Exp. Med. 187-685). These observations demonstrate that HSPs can be efficient CD8⁺T cell response eliciting adjuvants.

The tumors naturally express more HSP molecules (HSP70, HSP65, HSP90 for example) than normal tissues and the number of HSP molecules expressed can be markedly augmented in cases of stress (heat, shock, irradiation, surgery, chemotherapy,...). The immune system is normally tolerant to tumor HSP due to its identity to HSP expressed in normal tissue.

What can make a protein antigenic is a mutation in its sequence allowing its recognition by the immune system. Normally such mutation is not found in HSP, however a rare HSP70 antigen encoded by a mutated form of HSP-70.2 gene was recently found in a patient with renal cell carcinoma (C. Gaudin et al. 1999. A hsp 70-2 mutation recognized by CTL on a human renal cell carcinoma. The Journal of Immunology 1730-1738). This mutated form of HSP70 was specifically recognized by cytotoxic T lymphocytes. A point mutation on the conserved HSP structure induced a dramatic increase in immunogenicity. The sequence of the immunogenic peptidedecamer was described allowing obtention of mutated HPS for immunotherapy.

However, a general stress would induce HSP in tumors as well as in normal cells.

All the tentative antitumoral treatment by stress induction on tumor cells did not induce specific immune response to HSP nor significative tumor response.

One of the aims of the invention is to provide a process for breaking the immunotolerance to HSP.

Another aim of the invention is to provide a process to enhance specifically HSP in a tumor or infectious area, to direct immune cytotoxic responses only to tumor or infected sites.

Another aim of the invention is to provide a process to achieve optimal antitumoral or antiinfectious response.

In a broad embodiment, the invention relates to lymphocytes, having the property of recognizing cells of a patient in which the pericellular membrane expression of HSP or presentation of HSP epitopes has been induced and increased with respect to the normally occurring HSP expression level in the patient's body, and corresponding to an overexpression of HSP, particularly at a level of at least about 10 times the normally occurring level.

In the text, the term "HSP" corresponds to peptidic compounds, comprising at least an amino acid sequence having at least 80 % homology with the amino acids between the positions 286 and 294 of HSP70. The said peptidic compounds comprise isoleucine, leucine, valine, alanine, glycine or phenylalanine on position 293, and more particularly isoleucine.

It is to be noted that the term "overexpression" also designates overpresentation, which corresponds to an increase of HSP epitopes presented on the membrane.

In particular, HSP which is used in the invention comprises at least one of the following sequences :

- SEQ ID n° 1 : 286-SLFEGIDIY-294
- SED ID n° 2 : 286-SLFEGIDIYT-295
- SEQ ID n° 3 : 286-SLFEGIDFYT-295

The expression "peptidic compounds" designates an entity constituted by at least a HSP70 peptidic fragment above defined or by a succession of said peptidic fragments, and having possibly one or several other elements different from natural amino acids.

These elements have the aim to protect chemically or physically said peptidic fragments and/or to favour their absorption by organism, and/or their administration, and/or their biodisponibility.

For example, this protection enables the peptides to reach their targets without undergoing the action of several proteases which are present in the organism. Such chemical modifications can also increase the affinity of an antigenic peptide for the molecule HLA-A2 and enable an increased efficiency of in vivo vaccine. Said elements can be for example :
- chemical protecting groups known by the man in the art,
- chemical groups improving the efficiency of the vaccine *in vivo,*
- lipids or fatty acids which are covalently bound to the peptidic fragments to form lipopeptides,
- a carrier protein of said peptidic fragments presenting restriction sites and enabling the route of the inctact peptide fragments to their site of action in organism.

The expression "lymphocytes having the property of recognizing cells "designates lymphocytes having receptors with high affinity for antigens expressed or presented on the membranes of said cells.

HSP is expressed intracellularly and almost not on the membrane of non transformed cells, i. e non tumor cells or non infectious cells.

In the case of transformed cells, i.e tumor or infectious cells, the amount of HSP expressed or presented on the membrane is increased, but not in an amount sufficient for HSP to be recognized by the immune system and not under a form which, in itself, makes it antigenic, as it is a conserved protein.

The expression "normally occuring level of HSP" corresponds to the level of HSP expressed by tumor or infectious cells.

The expression "induced and increased" means triggering of an active response with de novo synthesis of HSP and expression on the cell membrane after a stress.

The expression "overexpression of HSP" means expression of HSP higher than the normally occuring level of HSP above defmed. Membrane HSP expression level can be determined by classic immunological methods such as ELISA, FACS analysis, immunohistology or Western blotting.

The evidence that lymphocytes can recognize cells having overexpression of HSP can be shown by the cytotoxic activity that T lymphocytes express against these cells (for example Cr⁵¹ release assay) or by the increased proliferation of the lymphocytes recognizing the HSP antigen.

An advantageous embodiment, the invention relates to lymphocytes, which are T lymphocytes specific for cells having an overexpression of HSP on their pericellular membrane, and more particular T8 lymphocytes able to lyse said cells which have an overexpression of HSP, or T4 helper lymphocytes, and not to lyse cells with normally occurring expression of HSP.

"T8 lymphocytes" designate cytotoxic T lymphocytes expressing CD8 membrane marker and having receptors for HSP polypeptidic sequence.

"T4 helper lymphocytes" are helper T lymphocytes which express CD4.

These lymphocytes are specific for cells having an overexpression of HSP on their pericellular membrane, specificity meaning that they engage in high affinity, interactions and can be determined by classic immunological methods such as ELISA, FACS analysis or immunohistology.

The lymphocytes of the invention are such that they do not lyse cells with normally occuring expression of HSP, which means that they do not interact with cells having no or very low HSP on their membrane.

According to another advantageous embodiment of the invention, the lymphocytes, which are B lymphocytes, secrete antibodies recognizing specifically cells which have an overexpression of HSP on their pericellular membrane.

The B lymphocytes which are specific for HSP (presenting adequate receptor) will produce anti HSP antibodies (IgG/IgM)

According to another advantageous embodiment, the cells which have an overexpression of HSP are tumor cells or infectious cells, particularly virus infected cells.

In the present text, tumor cells or infectious cells are also designated as transformed cells.

According to another advantageous embodiment, the overexpression of HSP is induced by physical of chemical means, to induce locally a stress responsible for an overexpression of HSP on target cells, and in particular the local stress is induced:
- by submission of the body to variable magnetic field after injection of magnetic beads, and particularly ferrofluids, coupled to antibodies recognizing target cells and accumulating in these target cells,
- or by laser beam targeting the target cells ,
- or by local surgery ,
- or by radiation therapy,
- or by local drug delivery (chemotherapy) or by starvation from essential nutrients or of growth factors, chemokines, and hormones, essential for rapid proliferation of target cells.

A variable magnetic field has the property of inducing agitation of target cells. Ferrofluid are very small magnetic beads in the range from about 5 nm to about 100 nm.They can be chemically coupled to antibodies against the target cells. Laser beam targeting is a local excitation of transformed cells by laser technology which results in stress.

Local surgery, radiation or local drug delivery or starvation produce local cell death apoptosis and stress.

The invention also relates to a process for preparing lymphocytes as above defined, comprising the steps of:
- loading antigen presenting cells with a mutated immunogenic form of HSP or cDNA coding for a mutated immunogenic form of HSP, to obtain antigen presenting cells presenting the mutated immunogenic form of HSP ;
- induction of a primary immune response against the mutated immunogenic form of HSP to obtain lymphocytes specific for said mutated immunogenic form of HSP and recognizing non mutated form of HSP ;
- induction of a secondary immune response against cells induced to have an overexpression of HSP on their membrane, to obtain an amplification of lymphocytes recognizing said cells with an affinity with respect to non mutated HSP overexpressed on said cells, with said affinity being increased compared to that obtained in the primary immune response.

APCs are loaded with mutated HSP, which comprises phagocytosis or pinocytosis of mutated HSP, which lead to intracellular degradation (processing) and expression of epitopes on the APC membrane in association with MHC molecules. As a consequence, APC will specifically present mutated HSP epitopes to lymphocytes.

The preparation of the lymphocytes of the invention implies a binary immune response comprising :
- a first immune response leading to lymphocytes recognizing mutated HSP and non mutated HSP with the synthesis of specific IgM/ IgG,
- a second immune response to amplify lymphocytes recognizing non mutated HSP, more precisely said second immune response being a result of a boost by induced overexpression of non mutated HSP which result in the synthesis of high level of Ig G and in the proliferation of specific T lymphocytes against HSP epitopes. It is to be noted that the lymphocytes have higher affinity for the target cells overexpressing HSP than those obtained further to the induction of the primary response.

In the present text, the expression "non mutated HSP " designates wild type HSP.

The affinity constant Ka of lymphocytes amplified in the secondary immune response varies from 10 ⁹ to 10¹¹ M ⁻¹ .

According to another advantageous embodiment, in the process of the invention, the induction of a primary response against the mutated immunogenic form of HSP comprises the following step :
∗ either injection to a patient of antigen presenting cells loaded with:
   - a mutated immunogenic peptide derived from HSP,
   - or a mutated immunogenic protein derived from HSP,
   - or cDNA coding for a protein containing said mutated immunogenic peptide or protein,
   - or a bacterial or viral vector coding for said mutated immunogenic peptide or protein, to obtain lymphocytes specific for said mutated immunogenic form of HSP and recognizing non mutated form of HSP ;
∗ or contacting antigen presenting cells, presenting the mutated immunogenic form of HSP, with lymphocytes from a patient, to obtain a mixture of antigen presenting cells and lymphocytes educated against the mutated immunogenic form of HSP and injection of said mixture to the patient.

According to another advantageous embodiment, in the process of the invention, the mutated immunogenic form of HSP consists in HSP presenting a point mutation in a peptide derived from HEAT Shock protein and particularly from HSP-70, and in particular the mutated immunogenic form of HSP contains or corresponds to one of the following peptides :

Ser Leu Phe Glu Gly Ile Asp Phe Tyr Thr
Ser Leu Phe Glu Gly Ile Asp Ile Tyr
Ser Leu Phe Glu Gly Ile Asp Ile Tyr Thr

According to another advantageous embodiment, in the process of the invention, the secondary immune response is induced against cells having an overexpression of HSP comprises physical of chemical methods to induce locally a stress responsible for an overexpression of HSP on target cells, and in particular the local stress is induced:
- by submission of the body to variable magnetic field after injection of magnetic beads and particularly ferrofluids coupled to antibodies recognizing target cells and accumulating in these target cell,
- or by laser beam targeting the target cells,
- or by local surgery,
- or by radiation therapy,
- or by local drug delivery (chemotherapy) or by starvation from essential nutrients or of growth factors, chemokines, and hormones, essential for rapid proliferation of target cells.

The invention also relates to the use of the process of the invention, for the treatment of cancer or intracellular infections.

The lymphocytes of the invention are useful for the treatment of cancer and infections, in which it is possible to induce overexpression of HSP.

The invention also relates to pharmaceutical compositions, containing lymphocytes as defined above and possibly antigen presenting cells specific for HSP, in association with a pharmaceutically acceptable vehicule.

The invention also related to a method for the treatment of cancer or of intracellular infections comprising
- vaccination as above defined, by which the tolerance of the organism to the tumor or to the virus associated HSP is broken using immunogenic mutated form of HSP and induction of a stress on the target tumoral or intracellular infected cells. The transformed cells are not efficiently recognized by the immune system, which means that there is a tolerance.

A mutation of the antigen (HSP) makes it immunogenic, and the tolerance towards the wild HSP is broken.

As to the induction of stress, it is done by physical or chemical means which result in stressed cells and expression of stress proteins.

A more detailed description of the invention is given hereafter.

The induction of tumor rejection by host cellular and humoral immunity is achieved by two steps: Immunization against a mutated form of Heat Shock Protein (HSP) combined with specific means of shocking tumor cells causing HSP overexpression.

First, the patients bearing tumors are immunized with recombinant mutated-HSP or mutated HSP70.2 decapeptide. This immunization is achieved for example with mutated peptides plus adjuvant, or with patient autologous dendritic cells pulsed with the m-peptide, or by injection with viral vectors expressing the m-peptide.

Second, HSP-70 overexpression in specifically induced in the targeted tumor cells. Therefore, magnetic nanoparticules (Ferrofluid) coupled to anti-tumor surface antigen Fab antibody are injected to the patients. Then the patient is submitted to a variable magnetic field of high intensity and frequency. This causes local temperature increase of several degrees in tumor cells covered with ferrofluids, resulting in overexpression of heat shock proteins and particularly of HSP-70. The tumor cells therefore become the target of a very strong secondary immune response against HSP overexpressed on tumors.

This approach can also be extended to patients infected with intracellular pathogens and particularly viruses. In this case, the antibodies used are directed against a viral antigen expressed by the infected cells. After an identical two steps treatment, the infected cells are recognized and killed by the elicited specific immune response of the patient.

### EXAMPLES

### 1) Examples in vitro

Transformed human B cells are obtained after infection of peripheral blood mononuclear cells with Epstein Barr Virus (EBV). These cells are selected from HLA A2 patients and express B cell receptor (BCR). They are maintained in culture in RPMI 1640 medium (Lifecell Technology, GB) supplemented with 10% foetal calf serum.

Ferromagnetic nano particules (ferrofluid of 1 to 20 nm) are coupled to anti-BCR antibodies (anti-BCR antibodies, Immunotech, Fr) in phosphate buffered saline containing 1 % human serum albumin.

The infected B cells (5-10⁶) are incubated for one hour on ice with an excess of antibody coated ferrofluid. Unbound ferrofluid is eliminated by washing and the cells are subjected to a magnetic field. This magnetic agitation induces overexpression of stress proteins and in particular of HSP-70 on the surface of B cells. This induction of HSP-70 is measured by Western Blot analysis using monoclonal antibodies against the constitutive and inducible forms of HSP-70.

A mHSP-70 specific T cell clone is obtained by the following technique :PBMC (10⁷) and MAC-DC (10⁷) loaded with the mutant peptide of HSP-70 (Triebel, IGR) are seeded in 6 well plates in 5ml complete RPMI containing 10 UI/ml rIL-2, 50 UI/ml rIl-7 (Sanofi) and 10 UI/ml IL 12 -Genetics Institute). On day 7^{th} of the culture, fresh MAC-DC and fresh medium are added.

After culturing for 3 weeks, lymphocytes are cloned by limiting dilution in 96 wells containing complete RPMI with 30 IU rIl-2 and 2% TCGF and 10⁵
irradiated EBV-transformed autologous cells. Clones are split twice a week. After 3 week expansion, clones are tested for their lytic potential.

The T cell clones obtained are able to lyse specifically the B cells overexpressing HSP-70 after submission to the magnetic field. In contrast B cells not submitted to the magnetic field are not lysed by the T cell clone specific for mHSP70. The EBV transformed B cells loaded with the mutated peptide from HSP-70.2 (SLFEGIDFYT) or with the wild type HSP-70.2 peptide (SLFEGIDIYT) are recognized and lysed by the specific T cell clone.

This example shows that one can obtain lymphocytes having the property to recognize specifically transformed human cells in which the membrane expression of HSP has been induced and increased by stress with respect to the same cells which have no induction of HSP overexpression.

The sequence of specific induction of anti-mHSP T cells and the submission of target human cells to stress (and HSP overexpression as a result) allows immune recognition and lysis.

### 2) Obtaining tumor regression in experimental murine model

HHD Mice (Kb-, Dd-, b2m-, HLA-A2 HDD+) are immunized against the mutated peptide of HSP-70.2 (SLFEGIDFYT) by subcutaneous injection of 10⁶ dendritic cells (differenciated from murine bone marrow in the presence of GM-CSF and IL-13) loaded with mutated mHSP-70 protein. After one week, they are inoculated with EL4 tumor (EL4 S3 RobHHD, Db-, Kb-, HLA A21+) transfected with BCR gene.

After another 4 days mice receive intravenous injection of ferrofluid coupled to anti-BCR monoclonal antibodies. Five mice out of 10 are submitted to magnetic field. Only the animals previously immunized and exposed to the magnetic field show a marked (> 50%) tumor regression. In contrast, control mice not immunized against mutated HSP70 or immunized but not exposed to magnetic field experience normal tumor growth.

## Claims

1. Lymphocytes, having the property of recognizing cells of a patient in which the pericellular membrane expression of HSP or presentation of HSP epitopes has been induced and increased with respect to the normally occurring HSP expression level in the patient's body, and corresponding to an overexpression of HSP, particularly at a level of at least about 10 times the normally occurring level.

2. Lymphocytes according to claim 1, which are T lymphocytes specific for cells having an overexpression of HSP on their pericellular membrane, and more particular T8 lymphocytes able to lyse said cells which have an overexpression of HSP, or T4 helper lymphocytes, and not to lyse cells with normally occurring expression of HSP.

3. Lymphocytes according to claim 1, which are B lymphocytes, secreting antibodies recognizing specifically cells which have an overexpression of HSP on their pericellular membrane.

4. Lymphocytes according to anyone of claims 1 to 3, wherein the cells having an overexpression of HSP are tumor cells or infectious cells, particularly virus infected cells.

5. Lymphocytes according to anyone of claims 1 to 4, wherein overexpression of HSP is induced by physical of chemical means to induce locally a stress responsible for an overexpression of HSP on target cells, and in particular the local stress is induced:
- by submission of the body to variable magnetic field after injection of magnetic beads, and particularly ferrofluids, coupled to antibodies recognizing target cells and accumulating in these target cells,
- or by laser beam targeting the target cells,
- or by local surgery ,
- or by radiation therapy,
- or by local drug delivery (chemotherapy) or by starvation from essential nutrients or of growth factors, chemokines, and hormones, essential for rapid proliferation of target cells.

6. Process for preparing lymphocytes according to anyone of claims 1 to 5, comprising the steps of:
- loading antigen presenting cells with a mutated immunogenic form of HSP or cDNA coding for a mutated immunogenic form of HSP, to obtain antigen presenting cells presenting the mutated immunogenic form of HSP ;
- induction of a primary immune response against the mutated immunogenic form of HSP to obtain lymphocytes specific for said mutated immunogenic form of HSP and recognizing non mutated form of HSP ;
- induction of a secondary immune response against cells induced to have an overexpression of HSP on their membrane, to obtain an amplification of lymphocytes recognizing said cells with an affinity with respect to non mutated HSP overexpressed on said cells, with said affinity being increased compared to that obtained in the primary immune response.

7. Process according to claim 6, wherein the induction of a primary response against the mutated immunogenic form of HSP comprises the following step :
∗ either injection to a patient of antigen presenting cells loaded with:
- a mutated immunogenic peptide derived from HSP,
- or a mutated immunogenic protein derived from HSP,
- or cDNA coding for a protein containing said mutated immunogenic peptide or protein,
- or a bacterial or viral vector coding for said mutated immunogenic peptide or protein, to obtain lymphocytes specific for said mutated immunogenic form of HSP and recognizing non mutated form of HSP ;
∗ or contacting antigen presenting cells, presenting the mutated immunogenic form of HSP, with lymphocytes from a patient, to obtain a mixture of antigen presenting cells and lymphocytes educated against the mutated immunogenic form of HSP and injection of said mixture to the patient.

8. Process according to claim 7, wherein the mutated immunogenic form of HSP consists in HSP presenting a point mutation in a peptide derived from heat shock protein and particularly from HSP-70, and in particular the mutated immunogenic form of HSP contains or corresponds to one of the following peptides:

Ser Leu Phe Glu Gly Ile Asp Phe Tyr Thr
Ser Leu Phe Glu Gly Ile Asp Ile Tyr
Ser Leu Phe Glu Gly Ile Asp Ile Tyr Thr

9. Process according to anyone of claims 6 to 8, wherein the secondary immune response is induced against cells having an overexpression of HSP comprises physical of chemical methods to induce locally a stress responsible for an overexpression of HSP on target cells, and in particular the local stress is induced:
- by submission of the body to variable magnetic field after injection of magnetic beads and particularly ferrofluids coupled to antibodies recognizing target cells and accumulating in these target cell,
- or by laser beam targeting the target cells,
- or by local surgery,
- or by radiation therapy,
- or by local drug delivery (chemotherapy) or by starvation from essential nutrients or of growth factors, chemokines, and hormones, essential for rapid proliferation of target cells.

10. Use of the process according to anyone of claims 6 to 9, for the treatment of cancer or intracellular infections.

11. Pharmaceutical composition, containing lymphocytes according to anyone of claims 1 to 5 and possibly antigen presenting cells specific for HSP, in association with a pharmaceutically acceptable vehicule.

12. Method of the treatment of cancer or of intracellular infections comprising
- vaccination according claim 6, by which the tolerance of the organism to the tumor or to the virus associated HSP is broken using immunogenic mutated form of HSP and induction of a stress on the target tumoral or intracellular infected cells.


### Annex to the application documents - subsquently filed sequences listing

LISTE DE SEQUENCES

<110> I.D.M. IMMUNO-DESIGNED MOLECULES

<120> NEW LYMPHOCYTES, A PROCES FOR PREPARING THE SAME AND
      THEIR USE IN THERAPEUTICS

<130> IOB 99BA IDM STRE

<140> EP99120484.3
<141> 1999-10-15

<160> 3

<170> PatentIn Ver. 2.1

<210> 1
<211> 9
<212> PRT
<213> Homo sapiens

<400> 1
Ser Leu Phe Glu Gly Ile Asp Ile Tyr
  1               5

<210> 2
<211> 10
<212> PRT
<213> Homo sapiens

<400> 2
Ser Leu Phe Glu Gly Ile Asp Ile Tyr Thr
  1               5                  10

<210> 3
<211> 10
<212> PRT
<213> Homo sapiens

<400> 3
Ser Leu Phe Glu Gly Ile Asp Phe Tyr Thr
  1               5                  10